(19) Europäisches Patentamt
European Patent Office
Office européen des brevets

(11) Publication number: **0 237 686**
**A1**

# (12) EUROPEAN PATENT APPLICATION

(21) Application number: **86400576.4**

(22) Date of filing: **18.03.86**

(51) Int. Cl.⁴: **C12N 15/00**, C12P 21/02, A61K 39/205, C12Q 1/70, C07K 15/00

The title of the invention has been amended (Guidelines for Examination in the EPO, A-III, 7.3).

(43) Date of publication of application: **23.09.87 Bulletin 87/39**

(84) Designated Contracting States: **AT BE CH DE FR GB IT LI LU NL SE**

(71) Applicant: **INSTITUT PASTEUR**
**25-28, rue du Docteur Roux**
**F-75724 Paris Cédex 15(FR)**
Applicant: **CENTRE NATIONAL DE LA RECHERCHE SCIENTIFIQUE (CNRS)**
**13 Quai Anatole France**
**F-75700 Paris(FR)**

(72) Inventor: **Tordo, Noel**
**14, rue Alexandre-Cabanel**
**F-75015 Paris(FR)**
Inventor: **Poch, Olivier**
**13, Route du Polygone**
**F-67000 Strasbourg(FR)**
Inventor: **Keith, Gérard**
**14 rue des Faisans**
**F-67800 Bischheim(FR)**
Inventor: **Ermine, Alain**
**58, rue Myrha**
**F-75018 Paris(FR)**

(74) Representative: **Gutmann, Ernest et al**
**S.C. Ernest Gutmann - Yves Plasseraud 67, boulevard Haussmann**
**F-75008 Paris(FR)**

## (54) DNA sequences derived from the rabies virus genome.

(57) The invention concerns DNA fragments belonging to the genome of the rabies virus, more particularly the fragments encoding the M1, M2 and L proteins. The fragments can be used for the production of hybridization probes and also for the production of corresponding polypeptides having immunological properties in common with the abovesaid proteins, by microorganisms transformed by said DNA fragments.

EP 0 237 686 A1

# DETERMINED DNA SEQUENCES DERIVED FROM THE RNA GENOME OF THE RABIES VIRUS AND CORRESPONDING PROTEINS ENCODED BY SAID DNA SEQUENCES, THEIR USES FOR IN VITRO DIAGNOSTIC PURPOSES AND THE PRODUCTION OF ANTIGENIC COMPOSITIONS

The invention pertains to determined DNA sequences derived from the RNA genome, more particularly cDNA sequences and the DNA recombinants, particularly vectors, modified by such DNA sequences in such manner that, when said DNA recombinants are introduced in suitable host cells in which said DNA recombinants can be replicated, the said DNA sequences can be expressed in the form of the corresponding proteins. The invention further relates to the proteins themselves, which can be purified and used for the production of immunogenic compositions.

Rabies virus is a bullet shaped virus containing a non segmented negative strand RNA genome. Rabies virion consists in a nucleocapsid core surrounded by a host derived lipidic enveloppe. The nucleocapsid core which is thought to present all elements for the viral transcription (Kawai, 1977), is composed of the genomic RNA (about 12 kb) associated with the nucleoprotein N (MW=58500), the phosphoprotein M1 (MW=39500) and the transcriptase L (MW=170000). Two membrane proteins are found in the enveloppe : the protein M2 (MW=25000) on the innerside and the transmembrane glycoprotein G (MW=70500) which presents antigenic spikes on the outside. As for the Vesicular Stomatitis Virus (VSV), another member of the Rhabdoviridae family, transcription of the rabies genomic RNA produces sequencially one leader RNA - (Kurilla et al., 1984) and five polyadenylated monocistronic mRNAs (Coslett et al., 1980 ; Holloway and Obijeski, 1980) defining the gene order along the genome from 3′ to 5′ : leader , N, M1, M2, G and L - (Flamand and Delagneau, 1978).

Despite their analogical genome organization and similar transcriptive and replicative strategy (Holloway and Obijeski, 1980), rabies virus and VSV exhibit notable differences in their biological activity. In partuclar VSV has a greater cell killing ability (Marcus and Sekellick, 1975) and causes much greater inhibition of host cell macromolecular synthesis by a mechanism involving the leader RNA (Weck et al., 1979). It was therefore interesting to compare the VSV genome whose nucleotide sequence has been widely documented (Rose, 1980 ; Rose and Gallione, 1981 ; Gallione et al., 1981 ; Schubert et al., 1984) with the rabies virus genome about which relatively little is known, except for the glycoprotein structure which has been determined by cloning of a cDNA copy of G mRNA (Anilionis et al., 1981 ; Yelverton et al., 1983 ; Malek et al., 1984).

Reference is made to the European patent application n° 94 887, to the French patent application n° 2 552 776 and the related certificate of addition n° 2 562 090. This earlier application referred to the cloning of a DNA sequence encoding the transmembrane glycoprotein G and to the production of immunogenic compositions containing said glycoprotein.

Rabies virus genome is a non segmented negative strand RNA (1) of approximately 12 Kb. It encodes one leader RNA at the 3′ end (2) and five monocistronic mRNAs (3, 4) corresponding successively to the nucleoprotein N, the phosphoprotein M1, the membrane protein M2, the glycoprotein G and the polymerase L (5). As it is the case for all non segmented negative strand RNA viruses, rabies genome is always found encapsidated into a nucelocapsid core including N, M1 and L proteins. M2 and G proteins are located in the viral envelope (6, 7). During the infectious cycle, the RNA genome in the nucleocapsid core is used sequentially as template for the transcription into monocistronic RNA(+) (leader RNA and mRNAs), and for the replication into genomes(+) also encapsidated (8, 9). These, in turn, will be replicated in genomes(-) themself encapsidated in the progeny virions. This multiplication system implies that the sequences located at the gene boundaries should be recognized as initiation and termination sites during the transcriptional phase, while they should be ignorized during the replication step. In the case of VSV and Sendaï virus, two prototypes of non segmented negative strand RNA viruses the extensive homology found at every gene junction allowed to deduce three consensus sequences involved in the transcriptional process (10, 11): the 3′stop of the mRNA with its (A)track polyadenylation signal, the intergenic region whose complement does not appear in the mRNA and the 5′start of the mRNA. A strong similarlity was observed between the consensus regions of the two viruses (11).

The invention stems from the cloning strategy disclosed hereafter of the genome of the rabies virus which enables other DNA sequences to be identified, more particularly those encoding the N, M1, M2 proteins. Reference is more particularly made to the drawings in which :

-figure 1 is illustrative of the cloning strategy which has been adopted and which will be described hereafter in a more detailed manner ;

-figure 2 shows the results obtained in two dimensional electrophoresis homochromatography after random hydrolysis (A) and acrylamide gel electrophoresis (B) of 3' labelled genomic RNA, for the sake of determining its sequence ;

-figure 3 shows Northern blot of total RNA (A) and a pattern of translation products of said RNA (B) from rabies PV infected or non infected cells ;

-figure 4 shows the sequence of 1 500 nucleotides at the 3' end of the rabies PV genome (presented as complementary DNA (+) sens) ;

-figure 5 diagrammatizes the conditions under which the 5' end of Nm RNA was mapped ;

-figure 6 shows an alignment of the PV and VSV Indiana nucleoproteins as effected using a computer program ;

-figures 7-9 provide the entire sequence of the non segmented negative strand RNA of the rabies virus.

More particularly, the invention is thus now concerned with the whole non segmented negative strand RNA shown in figure 2 as well as with the cDNA that can be derived therefrom.

Preferred sequences are those which encode full proteins, more particularly and respectively the nucleotidic sequences (with respect to the first nucleotide of the RNA leader, as shown in figures 7-9) :
-extending from nucleotide 71 (ATG) to nucleotide 1 421 (TAA) which encodes the nucleotide protein ;
-extending from nucleotide 1 514 to nucleotide 2 404, which sequence encodes the M1 protein ;
-extending from nucleotide 2 496 to nucleotide 3 102, which sequence encodes the M2 protein.

It also relates to sequences including that which extends from nucleotide 5 417 to nucleotide 5 500, and which encodes for the N-terminal portion of the L protein. It goes without saying that the invention also pertains to any nucleotidic sequence related to the preceding ones which may be obtained at least in part synthetically, and in which the nucleotides may vary within the constrainsts of the genetic code, to the extent where these variations do not entail a substantial modification of the polypeptidic sequences encoded by the so-modified nucleotidic sequences.

The above nucleotide sequences can be inserted in vectors, to provide modified vectors which, when introduced in the suitable cell host, are capable of causing the latter to transcribe and translate said DNA sequences to provide the corresponding proteins which can then be isolated from cellular extracts of the host. Obviously it is within the knowledge of the man skilled in the art to select the appropriate vectors, particularly in relation to the host to be transformed therewith. Vectors consist for instance of plasmids or phages which will be selected according to their recognized capability of replicating in the corresponding procaryotic cells (or yeast cells) and of allowing for the expression of the DNA sequence which they carry.

The invention also relates to DNA recombinants containing an insert consisting of a DNA sequence or part thereof, and suitably engineered to allow for the expression of the insert in eucaryotic cells, particularly cells of warm-blooded animal. Suitable DNA recombinants are genetic constructs in which said insert has been placed under the control of a viral or eucaryotic promoter recognized by the polymerases of the selected cells and which further comprise suitable polyadenylation sites downstream of said insert.

By way of example, the invention pertains to DNA recombinants containing any of the above mentioned inserts placed under the control of a promoter derived from the genome of the SV40 virus. Such DNA recombinants -or vectors-can be used for the transformation of higher eucaryotic cells, particularly cells of mammals (for instance Vero cells). The invention further pertains to portions of the above identified DNA sequences which, when inserted in similar vectors, are able to code for portions of the corresponding proteins which have immunological properties similar to those encoded by the full nucleotide sequences mentioned above. The similarity of immunological properties can be recognized by the capacity of the corresponding polypeptides produced by the relevant host to be recognized by antibodies previously formed against the proteins produced by the cells previously transformed with vectors containing the above mentioned entire DNA sequences.

The invention is not limited to the above mentioned sequences. It also relates to the intergenic sequences appearing in figures 7-9. Some of these are believed to be of significant importance for the control and regulation of the transcription and translation of the above specified sequences.

A significant advantage of this invention is that it provides DNA sequences derived from the RNA genome of the rabies virus which, in contrast with the genome itself, can be isolated in a form free of nucleoproteins. Thus the invention pertains more particularly to DNAs that are free of nucleoproteins. The invention also concerns entire cDNAs derived from the RNA genome, including the 3' end of the leader RNA. All the DNAs according to this invention can be used as a probe in hybridization experiments, for instance for the detection of the presence in a biological fluid from the patient of the invention by rabies virus. Techniques for carrying out these hybridization experiments are well known to the man skilled in the art.

The invention is more specifically related to probes consisting of mono stranded DNAs obtainable by denaturing of cloned double-stranded cDNA, by denaturation thereof and isolation of the opposite strands - (+) and strands (-).

Probes including the (+) strands are of value for the detection of the genome of the rabies virus and RNAs of the rabies virus which are not expressed, whereas the (-) strands are useful for the detection of the replicative forms of the virus.

Needless to say that the invention also pertains to all of said DNA sequences, when labelled by a suitable lable, i.e. a radioactive enzymatic or immunofluorescent label.

DNAs derived from the viral genome and which carry nucleotides modified by a chemical group which can be recognized by antibodies also form part of the invention. It is well known that such DNAs can be produced by nick-translation in the presence of nucleotides modified accordingly. These DNAs form particularly valuable hybridization probes which, when hybridized to a DNA preparation containing the complementary strand sought, can be detected by the above mentioned antibodies.

It already flows from the preceding discussion that the invention also pertains to the purified polypeptides N, M1 and M2 which are characterized by the peptidic structures which appear in figures 7-9 as well as to purified polypeptides containing the N-terminal portion of the L protein which appears in that figure. These polypeptides, when produced in a suitable host, can either be obtained from the cells, for instance after rupturing of their cell walls, or from the culture medium of said cells when excreted in said cell medium, depending on the cell DNA recombinant system which is used. The polypeptide obtained can than be purified by resorting to usual purification procedures. It should be understood that "purified" in the instant context means a level of purity such that, when electrophoresed in SDS-PAGE, the purified proteins yield a single detectable band, say by Western blot.

These purified polypeptides can in turn be used for the production of corresponding antibodies which can be used for diagnosing in vitro the presence of viral polypeptides in a biological fluid, particularly in a serum or tissue culture of a pateint. Like in the preceding instance, the invention relates to portions of the above defined polypeptides, particularly those which are recognized by the same antibodies or to the contrary are able to elicit in vivo the production of antibodies recognizing the full proteins.

A protein of particular interest is the M2 protein which is located in the envelope. The invention thus relates more particularly to immunogenic compositions containing the M2 protein in association with a vehicle used in the production of vaccines.

It must be understood that the invention relates also specifically to the particular peptides encoded by the DNA regions specifically referred to in the disclosure hereafter and which have been found of particular interest. Reference is again made to the peptides (which can be obtained by synthesis) which correspond to those encoded by the particular nucleotidic segments appearing in figures 7-9 and whose respective opposite extremities are recalled hereafter :

-peptide formed of aminoacides 139 to 170 (nucleotidic positions 1 928-2 023) ;

-peptide encoded by nucleotides extending from positions 2 760 to 2 816.

Preferred immunogenic compositions contain said M2 protein in admixture with other proteins such as the G protein or even the N and M1 proteins.

Other aspects of the invention will appear in the course of the disclosure of the cloning strategy referred to hereabove and more particularly of how the different portions of the RNA of the rabies virus were identified and cDNAs produced.

The starting virus used in the experiments disclosed hereafter is freely obtainable at the PASTEUR INSTITUTE of Paris, acting as a reference center of the World Health Organization (WHO). The strain concerned is the strain PV-12 used for the production of vaccines. It is also readily available at the American Type Culture Collection, under number ATCC VR-320.

The cloning of the rabies genome was undertaken by priming with an octadeca nucleotide complementary to the 3′ end. Here we describe cDNA clones corresponding to the 3′ extremity of the rabies genome including the leader RNA and the protein genes. The 5′ transcription initiation site of NmRNA was deduced from S1 protection experiments. The nucleotide sequence is compared with the corresponding region of VSV and with the 3′ end of the challenge rabies virus strain (CVS) genome recently published (KURILLA et al., 1984).

In order to define essential regions for the transcription and the replication of the rabies virus, we have undertaken the cloning of the RNA genome. cDNA clones have been obtained by walking along the genome using two DNA primers. From the nucleotide sequence, determined up to the beginning of the L protein gene, the aminoacid sequences of the N, M1, M2 and G proteins have been deduced. The consensus 5′ start and 3′ end of each rabies mRNA as well as the intergenic regions have been characterized in the non coding regions. All these regions are compared with other unsegmented negative strand RNA genomes.

cDNA clones of the rabies RNA genome have been obtained using two DNA primers : the first one was complementary to the 3′ extremity of the RNA genome and the second one was deduced from the nucleotide sequence of the M2 gene end. Following the strategy described in figure 1, we have determined the nucleotide sequence of 5 500 bases covering the 3′ moiety of the rabies genome (figures 7-9). The sequence contains four major non overlapping open reading frames corresponding respectively to the N, M1, M2 and G proteins. Other open reading frames either on the plus or the minus trand, never consist of more than 100 codons.

## RESULTS

### Cloning strategy of the 3′ region of the rabies genomic RNA

With the aim to establish the primary structure of the rabies genome, we have determined the 3′ terminal sequence of the genomic RNA. The 12,000 nucleotide long RNA was isolated by electrophoresis on low-melting point agarose gel and 3′ end-labelled as described in Methods. Sequence determination was performed using three different techniques : (1) the 3′ terminal nucleotide was identified as Up by thin layer chromatography (data not shown) ; (2) the sequence up to nucleotide 10 was determined by two-dimensional electrophoresis homochromatography (Figure 2A) ; (3) the sequence from nucleotide 7 to nucleotide 39 was determined by RNA chemical sequencing (Figure 2b). From the RNA sequence, we have synthesized a 18-mer DNA compelmentary to the 3′end to prime cDNA synthesis. cDNA-RNA hybrids or double stranded cDNA were inserted in pBR322 plasmid vectors.

To identify rabies recombinant clones, cDNA inserts were hybridized with RNA from infected or non infected BHK-21 cells. Figure 3A shows the results of Northern blot hybridization experiments with one of the clones (pRB43) (800 bp). The blot of infected cell total RNA (lane 2) gace a single additional hybridization band corresponding to a size of 1600 nucleotides when probed with ($^{32}$P)nick-translated insert. This is in agreement with the length expected for the rabies NmRNA (Holloway and Obijeski, 1980). In order to show that this mRNA effectively encodes the N protein, pRb43 plasmid DNA was used in hybridization selection experiments. Hybridized mRNA was eluted and in vitro translated in a rabbit reticulocyte lysate. Comparison of lames 2 and 3 of Figure 2B shows that the pRb43 insert selects an mRNA in infected cell extracts which directs the synthesis of a protein comigrating with the rabies N protein of PV strain (lanes PV). Furthermore, the translation product is immunoprecipitated by a mixture of 3 monoclonal antibodies directed against N protein (lane 4).

### Sequencing studies of cDNA clones, mapping of the 5′start of NmRNA

Two overlapping recombinant clones were used for sequence determination according to the strategy outlined in Figure 3. The resulting sequence, presented as DNA(+) sense, was determined until residue 1500. The 39 terminal nucleotides, complementary to the 3′extremity of the viral RNA, allow us to define the polarity of the insert.

Only one open reading frame uninterrupted until residue 1421 could be obtained, the other two contain multiple stop codons. Two ATG codons at position 41 and 71 respectively are potential initiation codons. In order to determine which was used, we carried out S1 protection experiments to define the transcription initiation site of NmRNA. As shown in Figure 5, the protected probe maps around residue $A_{90}$ (genomic sense). Because the first 32 nucleotides of each sequencing reaction belong to the cloning system - (universal primer and M13mp701 cloning sites) and are sensitive to nuclease S1 digestion, the transcription of NmRNA begins around residue $U_{59}$ (genomic sense). This result is in agreement with the previous obervation that leader RNA species of 55 to 58 nucleotides long is encoded by the 3′ extremity of the rabies genome (Kurilla et al., 1984). Thus, the nucleoprotein must be initiated at position 71.

The deduced amino acid sequence (Figure 4) consists of 450 residues. Consistent with the VSV nucleoprotein (Rose and Gallione, 1981), it has a small excess of basic amino acids and a calculated molecular weight (50550) slightly lower than the apparent molecular weight estimated from polyacrylamide gel electrophoresis (58500) (Holloway and Obijeski, 1980). The deficiency in CG dinucleotides, widely reported among negative strand RNA viruses, is also observed along the rabies NmRNA and results in a clear bias against CGN, NCG and NNC-GNN codons.

## DISCUSSION

All non segmented negative strand RNA viruses use the same basic mechanism for the multiplication : the genomic RNA (-) is first transcribed into monocistronic RNAs (+) (leader and mRNAs), then it is replicated into a complete genome (+) which will serve as template for the synthesis of novel genomic RNAs (-). Comparative studies between the VSV and rabies genomes can allow us to define more precisely the sequences involved in the transcription and replication. Since the RNA genome is always found associated with the nucleoprotein in the Rhabdovirus genus, we can expect to characterize in the N protein, the segments which are important for the formation of the nucleocapsid.

The sequence reported here includes two transcriptional units : the leader RNA and the nucleoprotein mRNA.

### The leader RNA

The leader RNA is a 3' transcription product which has been known for a long time in VSV (Colonno and Banerjee, 1976) and more recently demonstrated in the CVS strain of the rabies virus (Kurilla et al., 1984) (Figure 4). The 3' location of the leader region implies that such essential events as the initiation of transcription and replication (Emerson, 1982), the switching between these two functions (Blumberg et al. 1981) and the initiation of encapsidation (Blumberg et al., 1983) take place here. Kurilla has already shown some common features between VSV and rabies leader RNA : they are of similar length (about 50 nucleotides) with a high content of A residues (50 %), they both initiate with the same trinucleotide (ACG) which seems to be conserved throughout the Rhabdoviridae family (Giorgi et al., 1983) and have a very homologous 3' end. Our results on the rabies PV strain strengthens the idea of such a relationships. Nevertheless, comparison of the PV and CVS strains indicates that certain sequences are less important then had previously been thought. For example, the hexanucleotide 3' $_{14}$UUGGU$_{19}$5' (genomic sense) is present in the CVS strain as throughout the Vesiculovirus genus (one nucleotide upstream) where it is thought to be implicated in the RNA synthesis initiation (Giorgi et al., 1983). Since this hexanucleotide is absent from the PV strain due to one U/C change at position 15 and one G/A change at position 17, it is uncertain that it plays the same role in the rabies genome. Furthermore the two rabies strains show 11 mismatches between positions 1 and 180 (6 %). It is of interest to observe that this divergence is mainly located in the leader region (8 differences = 14 %) while only two silent substitutions are found in the nucleoprotein coding region (2.5 %). Consequently, the leader region appears to be an area of rapid evolution in the rabies virus genome. sequence studies of other rabies strains should permit us to better elucidate the variation in the leader region and to characterize the essential sequences.

### The nucleoprotein

Using the computer program of Lipman et Wilbur (1983), we have searched in protein data banks for sequences which may have homology with the rabies nucleoprotein. The only very significant homology - (score 4,4) was found with the nucleoprotein of VSV. Using the same program, the previously reported homology between the glycoproteins of the two viruses (Rose et al., 1982), is lower (score 3,3). Figure 6 shows one optimal alignment between rabies PV and VSV Indiana N protein. The invariant amino acids are not uniformly located along the sequence but some regions seem more conserved such as between amino acids 72 and 112 (41,5 %), 140 and 150 (54,5 %), 225 and 247 (39 %), 268 and 302 (48,5 %). In this last region, a stretch of 10 but one consecutive invariant amino acids is particularly remarkable (293 to 302). It is difficult to assign a function to these conserved regions on the sole consideration of sequencing data. Nevertheless, it is tempting to suggest that they are involved in the interaction of the nucleoprotein with the RNA genome.

### The flanking gene and intergenic sequences

Intergenic and flanking gene sequences have already been determined for VSV (Rose, 1980). Three consensus sequences were characterized in the non protein coding regions : the 5' transcription initiation site, the intergenic segment whose complement does not appear in the mRNA and the 3'end of mRNA with its polyadenylation signal $(A_7)$ (Table I).

S1 mapping protection experiments demonstrate the NmRNA transcription initiates around position 59 wuth the pentanucleotide 5′ AACAC 3′. It differs slightly from the canonical pentanucleotide 5′ AACAG 3′ of the VSV 5′transcription initiation site. Interestingly, such a VSV consensus site, but one nucleotide, appears in the middle of the leader RNA of the rabies PV strain (position 27) 14 nucleotides upstream from the $^{41}$ATG codon in the open reading frame. Although this structure could be a putative initiation site of NmRNA, S1 mapping experiments do not show any detectable transcription start at position 27 (Figure 5). On the other hand, the pentanucleotide 5′ AACAC 3′ beginning the rabies NmRNA, has also been involved in vitro in the initiation of one of VSV intracistronic transcription event (Schubert et al., 1982). Downstream from the N protein coding sequence, this pentanucleotide also appears in position 1485 and we therefore postulate that it represents the transcription initiation site of the MlmRNA. This assumption is strengthened by the finding of the sequence 5′ATG(A)$_7$3′ three nucleotides upstream (position 1473 to 1482), closely similar (one nucleotide less) to the consensus 3′ end of VSV mRNAs. This sequence must represent the polyadenylation signal of rabies NmRNA. Furthermore, the sequence 5′ ATATC 3′ beginning 21 nucleotides upstream from the MlmRNA transcription start site, looks like the promoter sequence preceding each VSV mRNA (Rose, 1980). However, in contrast to VSV, a similar promoter sequence for the NmRNA is absent in the rabies leader region. Finally, the 3′ stop of NmRNA and the 5′start of M1mRNA define a two nucleotides long untranscribed region (GA in the genome) identical to most of VSV intergenic segments. All these remarks are summarized in Table I.

The major conclusion of these results is that VSV and rabies virus are homologous only in limited regions. Despite a wide divergence of the nucleoprotein sequence, some stretches possibly involved in the interaction with the genomic RNA are highly conserved. Furthermore, as a result of the identical multiplication mechanism of these non segmented negative strand RNA viruses, a high conservation of transcriptional start and stop signals is observed. There is a strong homology between the 3′ end of the rabies NmRNA and the corresponding consensus sequence of VSV genes while the 5′ start sites are less homologous. Nevertheless, an extensive comparison of rabies N and M1 mRNA 5′ start sites, indicates that they both initiate with the sequence 5′ AACACCPyCT, Py being T and C respectively. Further sequence analysis of the genome is needed to elucidate a rabies 5′transcription start consensus sequence.

The different regions of the 3′ region of the rabies genomic DNA (figures 7-9).

Using SI nuclease protection experiments, we have previoulsy located the 5′ start of the NmRNA around position 59 on the rabies genome as discussed above. This was in agreement with another report of a 3′ leader RNA transcription product from 55 to 58 nucleotides long (2). Consequently, the first open reading frame encoding the nucleoprotein N, extends between the ATG codon in position 71 and the TAA codon at position 1421. The 450 amino acids N protein (50,5 kD) as well as the leader RNA sequence have been extensively studied in a previous report (Tordo et al., submitted).

The second open reading frame (1514 ATG to 2404 TAA) encodes the M1 phosphoprotein. The hydrophaticity profile along the deduced 290 amino acids sequence (14) reveals that it is the most hydrophilic protein among the four presented here. Hydrophilic residues are dominant in the first two $NH_2$ third of the protein. A particularly hydrophilic region is observed between amino acids 139 and 170 (position 1928 and 2023). Interestingly this region contains 13 of the 30 serine and threonine presumptive phosphorylation sites of the M1 protein. Since phosphate residues of the corresponding NS protein of VSV have been shown to be mostly anchored in the hydrophilic N terminal moiety (17, 18), it is possible that some of these serine and threonine residues could be used as phosphateacceptors in the rabies M1 protein.

The following two open reading frames code for the proteins present in the host-derived lipidic envelope of the virion. The sequence encoding the M2 protein extends from position 2496 to 3102. This corresponds to a 202 amino acid polypeptid with a 23 Kd MW. The sequence exhibits a central segment of 19 amino acids (position 2760 to 2816) very rich in hydrophobic residues. The hydropathicity program of Kyte et al. - (14) even predicts that this region has a high probability of being membrane bound. This is of note since it has been shown that in the case of negative strand RNA viruses, the membrane M protein is located in the inner side of the lipidic envelope (19, 6, 7) and could therefore interact both with the lipid bilayer and the ribonucleoprotein core (20). For VSV this interaction was shown to be at least partially hydrophobic (20, 21) although VSV M protein amino acid sequence does not show any long hydrophobic segment (22).

The transmembrane glycoprotein G responsible for the induction and binding of virus neutralysing antibodies (23), consists of 524 amino acids (58,5 kD without glycosylation residus) encoded between ATG in position 3318 and TGA in position 4980. The amino acid sequence presents two hydrophobic regions which have been previously described in the glycoprotein structure of ERA and CVS strains of rabies virus - (24, 25, 26). One consists of the 19 first amino acids of the $NH_2$ extremity which constitutes the signal peptid absent in the mature protein (27). The second hydrophobic segment is the transmembrane segment located in the C-terminal side of the protein and separating the hydrophilic cytoplasmic domain from the glycosylated spike. Four potential glycosylation sites appear in the spike. Three of these are shared by the ERA glycoprotein (Asn 37, 204, 319 ; position 3483, 4113, 4329 respectively) but an additional site appears at Asn 158 (position 3846).

Homology with other proteins.

We have recently reported that a significant homology concentrated in limited regions exists between the nucleopoteins of rabies virus and VSV (Tordo et al., submitted). Since another weaker homology was also detected with the nucleoprotein of Sendaï virus, we have proposed that a conservation of the nucleoprotein structure seems occuring through non segmented negative strand RNA viruses. In turn, we have searched the protein data banks as described in Methods, with the three others proteins presented here. No additional homology clearly emerging out of the background, could be encountered. Therefore, the nucleoprotein is the sole protein exhibiting some conservation. This would suggest that higher constraints have limited its divergence during evolution. According to its essential participation in the nucleoprotein core, it is possible that the amino acids involved in the maintenance of this structure, such as for example those which interact directely with the RNA genome, are more particularly conserved. Further comparison with other nucleoproteins of non segmented negative strand RNA viruses are needed to characterize more conserved regions.

In order to characterize a rabies mRNA 5'start consensus sequence, we have compared the nucleotide sequence at the 5'side of each protein initiator codon with the 5'extremity of the NmRNA determined by S1 nuclease protection experiments. The conclusion, summarized in Figure 3, indicate that a consensus mRNA start sequence of 9 nucleotides appears 12 to 30 residues upstream the translation initiation codon : the first four and the two last nucleotides are invariant ; in position 5 and 6 there are two pyrimidic residues but C is widely more frequent than T ; finally the seventh position is variable. This organization is very related to those of Sendaï virus and VSV where one or two variable nucleotides respectively separate two constant regions (10, 11). Rabies virus and VSV which share 5 invariant positions in the mRNA start consensus sequence, appear more related. Interestingly, in the only report of a rabies mRNA sequence dealing with GmRNA, authors, who have determined the sequence from position 3311, estimate that about 35 nucleotides from the 5' extremity are missing in the cDNA clone (22). According to the approximation of their estimation, this is in perfect agreement with our prediction of the 5'GmRNA start in position 3291, i.e. 20 nucleotides upstream, and strongly argues for the significance of the 5' consensus sequence. Further-more, we can observe that translation of rabies proteins is always predicted at the first ATG codon following the 5' consensus sequence as it is the case for all VSV and Sendaï virus mRNAs.

We have investigated if a consensus sequence also exist at the 3'mRNA extremities by comparing the genome regions extending from the translation stop codon of each protein to the 5'consensus extremity of the following mRNA with the 3' consensus sequences of VSV and Sendaï virus. These sequences invariably end with a polyadenylation signal ($A_7$ for VSV, $_5$ for Sendaï) which constitutes the initiation of the mRNA poly(A) tail. In the N-M1 and M1-M2 interregions, a seven(A) track is once encountered (position 1476 and 2469 respectively) and we therefore postulate that is represents the polyadenylation signal of N and M1 mRNA respectively. Interestingly, in M2-G and G-L interregions the situation there are two putative polyadenylation signals in position 3193 or 3279 and 4957 or 5357 respectively. If we consider the size of the M2 mRNA (4), the second seven(A) track (position 3279) should be more probably the initiator of M2 mRNA poly(A) tail. Nevertheless, we cannot exclude that the first polyadenylation signal may be used or represent a vestigial signal. In contrast, previous reports of GmRNA nucleotide sequence clearly imply the use of the first polyadenylation signal (position 4957) in the G-L intergenic region (22). An extensive comparison of the rabies mRNA 3'end allows us to construct a consensus sequence composed of two invariant nucleotides followed by a polyadenylation signal of currently 7 and exceptionnaly 8 (GmRNA) A residues. This organization is quite similar to that of VSV which presents two additional invariant nucleotides (Figure 6).

Therefore, our results show that all non segmented negative strand RNA viruses and more particularly Rhabdoviruses have conserved similar consensus sequences at each mRNA extremities despite a wide divergence in the protein structures. This finding outlights the importance of such sequences which do represent start and stop signals during the transcriptional process.

Intergenic regions.

There are defined between the 3' end of one mRNA and the 5'start of the following one. The intergenic regions of non segmented negative strand RNA viruses determined up to now, were very short, invariably composed of GA for VSV and GAA for Sendaï virus (10, 11).

Surprisingly, our results show that rabies intergenic regions are more variable both in length and nucleotide composition. Even thought that G nucleotide initiates each of them, N-M1, M1-M2, M2-G and G-L intergenes are 2, 5, 5 and 423 nucleotides long respectively.

The last value is unusually high. A more precise analysis of this G-L intergenic region reveals the following points : first, a nucleotide sequence relatively similar to rabies consensus mRNA start could be characterized 7 nucleotides downstream the GmRNA stop (position 4974) ; second, the distal poly(A) track in the G-L intergenic region (position 5357) looks like the polyadenylation signal encountered at the end of each rabies mRNA. Nevertheless, this intergenic region does not contain open reading frame longer than 20 codons. The functional significance of this unexpected intergenic region is unknown. But its localisation is particularly interesting to consider in the gene organization of the non segmented negative strand viruses. There are two families of such viruses. In contrast to Rhabdoviridae family which encodes only one glycoprotein, the Paramyxoviridiae family codes for an additional glycoprotein distinguishable by the N-terminal loction of its transmembrane segment. This glycoprotein gene which exhibit the haemaglutinin and sometime the neuraminidase activities, is located between the F fusion glycoprotein and the L polymerase, i.e. in the genomic region corresponding to the rabies G-L intergenic region. Therefore, it is tempting to propose that this intergenic region could represent a remnent gene in which the two sequences previously mentioned could correspond to start and stop consensus sequences at the 5' and 3' extremities.

## MATERIALS AND METHODS

### Virus growth and RNA purification

Pasteur strain of rabies virus (PV) was grown on BHK-21 cells as described (Ermine and Flamand, 1977). Virions were purified from culture supernatants by a method adapted from Arita and Atanasiu (1980). To isolate viral genomic RNA, purified virions were incubated with 100 µg/ml proteinase K ( Merck) in 1,5 % SDS, 100 mM Tris-HCl pH 7,5, 100 mM NaCl, 10 mM EDTA for 30 minutes at 37°C, followed by two phenol-chloroform extractions (vol/vol) and ethanol precipitation.

Extraction of total cellular RNA and selection of poly(A)$^+$RNA by oligo(dT)-cellulose chromatography were performed as described (Ermine and Flamand, 1977 ; Auffray and Rougeon, 1980).

### 3'end labelling and sequencing of genomic RNA.

Genomic RNA was 3'end labelled with ($^{32}$P)Cp and T4 RNA ligase (England and Uhlenbeck, 1978). Exhaustive hydrolysis occured in 15 % piperidin and the end nucleotide was determined by thin layer chromatography on cellulose. Partial hydrolysis was performed in distilled water for 2.5 hours at 90°C before two-dimensional electrophoresis homochromatography (Keith et al., 1984). Chemical RNA sequencing was carried out according to Peattie (1979).

### Cloning of the genomic RNA.

1 µg (about 0,25 picomoles) of purified genomic RNA was annealed to a 10 fold excess (mole/mole) of synthetic primer and transcribed into cDNA as described (Rougeon and Mach, 1977) with the following modifications : actinomycin D was omitted, 5 U/µl RNAsine (genofit) was added and incubation occured for 2.5 hours at 42°C. The ($^{32}$P) single stranded cDNA ($10^6$ cpm/µg) was either transcribed in double stranded cDNA (Gubler and Hoffman, 1983) or submitted to alkaline hydrolysis and gel filtration (G 75 sephadex), before rehybridized with freshly purified genomic RNA (ratio 1/5) during 2 hours at 65°C in Tris-HCl 20 mM pH 7.4, NaCl 300 mM, EDTA 1 mM, SDS 0.1 %. Hybrids cDNA-RNA or double stranded cDNA were treated with S1 nuclease, phenol extracted and fractionated on 1.5 % low-melting point agarose gel or 5-20 % sucrose gradient. Each class size was recovered and inserted in the PstI site of pBR322 plasmid vectors by the dC/dG tailing method (Rougeon and Mach, 1977). *E. coli* cells transformation was previously described (Roskam and Rougeon, 1979).

DNA sequencing, S1 protection experiments.

Endonuclease cleavage fragments of pRb43 and pRb28 plasmids were subcloned into M13 vectors (Messing et al., 1981) before enzymatic sequencing (Sanger et al., 1977). The 18-mer primer sequence was checked using the chemical sequencing method (Maxam and Gilbert, 1980). S1 protection experiments are described elsewhere (panthier et al., 1984).

Hybridization of cDNA with viral mRNAs.

Total cellular RNA was electrophoresed through a 1.2 % agarose gel containing formaldehyde and transferred to nitrocellulose filters (Schleicher & Schuell) (Thomas, 1980). Filters were hybridized with ($^{32}$P)-nick-translated cDNA probes (Rigby et al., 1977) overnight at 42°C in 50 % formamide. Conversely, filters were saturated with cDNA plasmids, hybridized with poly(A)$^+$RNA from infected cells and complementary mRNA was eluted.

*In vitro* translation of poly(A)±RNA.

Preparation of mRNA dependent rabbit reticulocyte lysates, translational conditions and analysis of translational products are described elsewhere (Auffray and Rougeon, 1980). Immunoprecipitations with monoclonal antibodies directed against the nucleoprotein were carried out according to Shih et al. (1970).

Computer analysis.

The protein data banks NBRF and PGtrans (Claverie et al., 1985) were searched using the program of Lipman and Wilbur (1983) with the following parameters : K-tuple size = 2, window size = 40 and gap penality = 2.

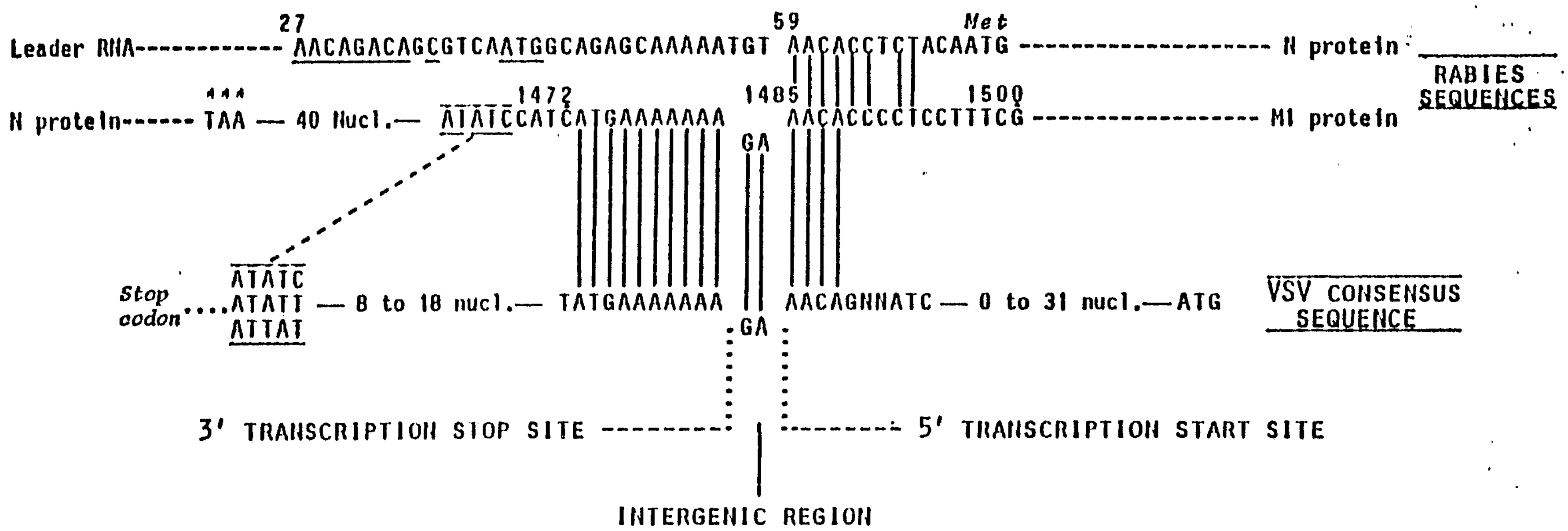


Table I: Intergenic and flanking gene regions.

There follows now the disclosure of the legends of the figures.

Figure 1 shows diagrammatically the conditions under which the complete primary structure of the sequence of the RNA genome (of PV rabies strain) was established. From the 3′ nucleotide sequence of the RNA genome, an octadecanucleotide complementary to the 3′ extremity was constructed and used to prime cDNA synthesis. Two overlapping recombinant cDNA clones hybridizing with the nucleoprotein mRNA - (NmRNA) were isolated and sequenced. The 1 500 first nucleotides of the rabies genome cover two transcriptional units : the leader RNA and the NmRNA which was shown to be initiated around residue 59 by S1 protection mapping experiments. Comparison between rabies PV and CVS strains up to residue 180 suggests a rapid evolution in the leader region. Studies of the sequence relationships between the 3′ regions of two Rhabdoviruses, rabies virus and Vesicular Stomatitis Virus (VSV), demonstrate that there is a segmented homology. Stretches of highly conserved aminoacids possibly involved in the interaction with the RNA genome were observed in the N protein despite a wide divergence in the remaining sequence. In addition, the high homology between the transcription start and stop signals reflects the conservation of a similar transcriptional mechanism in these two non segmented negative strand RNA viruses.

Figure 2 :

3′ labelled genomic RNA was analysed (A) by two-dimensional electrophoresis homochromatography after random hydrolysis and (B) by 20 % polyacrylamide gel electrophoresis after specific chemical cleavages. The resulting sequence is

A

3'OH-UGCGAAUUGUUGGUCUAGUUUCUUUUUUGUCUGUCGCAG...5'

B

Figure 3 :

(A). $^{32}P$ nick-translated pRb43 insert was hybridized to a Northern blot of total RNA from rabies PV infected (lane 2) or non-infected (lane 1) BHK-21 cells.

(B). 12 % SDS polyacrylamide gel separation of in vitro translation products of mRNA selected by hybridization with pRb43 insert among poly(A)$^+$RNA of infected (lane 3) or non-infectd (lane2) BHK-21 cells. Lanes 1 and 4 represent the immunoprecipitable products from lanes 2 and 3 respectively, using a mixture of 3 monoclonal antibodies directed against N protein. Lanes PV show proteins of purified virus (PV strain).

Figure 4 :

The sequence of 1500 nucleotides at the 3′end of the rabies PV genome (presented as complementary DNA (+) sense) was established after subcloning restriction fragments of ppRb43 and pRb28 inserts into M13 vector and enzymatic sequencing. The direction and extent of nucleotide reading are indicated by solid arrows. The dotted arrow represents the extent of genomic RNA sequencing. The deduced N protein sequence is presented. Wavy lines indicate the leader RNA, NmRNA and M1mRNA transcription start as well as the 3′end of NmRNA. The two potential N protein initiation codons with their respective transcription initiation site, the NmRNA polyadenylation signal and the postulated M1mRNA initiation site are underlined. The putative promoter sequence of the M1mRNA is boxed. The mismatched nucleotides in the CVS strain relatively to the PV sequence are notified up to residue 180 (vertical arrow) (Kurilla et al., 1984).

Figure 5 : Mapping of the 5′ end of NmRNA.

The AluI fragment from nucleotide 342 (genomic numeration) of pRb43 insert to the proximal AluI site in pBR322, was subcloned into the HincII site of M13mp701. Sequence corresponding to the genome is boxed and N-protein-coding region is hatched. A and E stand for AluI and EcoRI sites respectively. Uniformly $^{32}P$ labelled signal stranded DNA probe of genomic sense (DNA -) was synthesized. 5,$10^4$ cpm was hybridized with 1 µg or 4 µg of poly(A)+RNA from BHK-21 infected cells (lanes 2 and 3 respectively or non infected cells (lanes 4 and 5 respectively) and digested with S1 nuclease. $10^3$ cpm of undigested probe is shown in lane 1. The protected probe position is indicated with respect to enzymatic sequencing tracks (lanes A, C, G, T). Poly d(C) track constitutes the end of the pRB43 insert sequence. The wavy line deginates the transcription start point.

Figure 6 :

Alignment of the rabies PV and VSV Indiana nucleoproteins using the computer program of Lipman and Wilbur (1983) with the K-tuple size = 1, window size = 40 and gap penality = 1 parameters. Four particularly conserved regions are boxed.

There follows finally the list of references to the prior art, which is incorporated herein by reference. It must be understood that the contents of all of the citations referred to herein must be deemed as forming part of the disclosure to the extent necessary for the full understanding of the invention. The references are either introduced by names or by numbers according as they have been referred to in the course of the preceding disclosure.

REFERENCES

Anilionis,A., Wunner,W.H. and Curtis,P.J. (1981) Nature 294, 275-278.
Arita,M. and Atanasiu,P. (1980) Ann. Virol. (Inst. Pasteur) 131, 201-215.
Auffray,C. and Rougeon,F. (1980) Eur. J. Biochem. 107, 303-314.
Blumberg,B.M., Leppert,M. and Kolakofsky,D. (1981) Cell 23, 837-845.
Blumberg,B.M., Giorgi,C. and Kolakofsky,D. (1983) Cell 32, 559-567.
Claverie,J.M., Sauvaget,I. and Bougueleret,L. (1985) Biochimie 67, 437-443.
Colonno,R.J. and Banerjee,A.K. (1976) Cell 8, 197-204.
Coslett,G.D., Holloway,B.P. and Obijeski,J.F. (1980) J. gen. Virol. 49, 161-180.
Emerson,S.U. (1982) Cell 31, 635-642.
England,T.E. and Uhlenbeck,O.C. (1978) Nature 275, 560-561.
Ermine,A. and Flamand,A. (1977) Ann. Microbiol. (Inst. Pasteur) 128, 477-488.
Flamand,A. and Delagneau,J.F. (1978) J. Virol. 36, 133-142.
Gallione,C.J., Greene,J.R., Iverton,L.E. and Rose,J.K. (1981) J. Virol. 39, 529-535.
Giorgi,C., Blumberg,B.M. and Kolakofsky,D. (1983) J. Virol. 46, 125-130.
Holloway,B.P. and Obijeski,J.F. (1980) J. gen. Virol. 49, 181-195.
Gubler, U. and Hoffman,B.J. (1983) Gene 25, 263-269.
Kawai,A. (1977) J. Virol. 24, 826-835.
Keith,G., Pixa,G., Fix,C. and Dirheimer,G. (1984) Biochimie 65, 661-672.
Kurilla,M.G., Cabridilla,C.D., Holloway,B.P. and Keene,J.D. (1984) J. Virol. 50, 773-778.
Lipman D.J. and Wilbur W.J. (1983) Proc. Natl. Acad. Sci. USA 80, 726-730.
Malek,L.T., Soostmeyer,G., Garvin,R.T. and James,E. (1984) in Chanock,R.M. and Lerner,R.A. (eds.), Modern Approaches to Vaccines, Cold Spring Harbour, pp. 203-208.
Marcus,P.I and Sekellick,M.J. (1975) Virology 63, 176-190.
Maxam,A.M. and Gilbert,W. (1980) Methods Enzymol. 65, 499-566.
Messing,J., Crea,R. and Seeburg,P.H. (1981) Nucl. Acids Res. 9, 309-321.
Panthier,J.J., Dreyfus,M., Tronik-Le Roux,D. and Rougeon,F. (1984) Proc. Natl. Acad. Sci. USA 81, 5489-5493.
Peattie,D.A. (1979) Proc. Natl. Acad. Sci. USA 76, 1760-1764.
Rigby,P.W.J., Dieckmann,M., Rhodes,C. and Berg,P. (1977) J. Mol. Biol. 113, 237-251.
Rose,J.K. (1980) Cell 19, 415-421.
Rose,J.K. and Gallione,C.J. (1981) J. Virol. 39, 519-528.

Rose,J.K., Doolittle,R.F., Anilionis,A., Curtis,P.J. and Wunner,W.H. (1982) J. Virol. 43, 361-364.
Roskam,W.G. and Rougeon,F. (1979) Nucl. Acids Res. 7, 305-320.
Rougeon,F. and Mach,B. (1977) J. Biol. Chem. 252, 2209-2217.
Sanger,F., Nicklen,S. and Coulson,A.R. (1977) Proc. Natl. Acad. Sci. USA 74, 5463-5467.
Schubert,M., Barminson,G.G., Sprague,J., Condra,C.S. and Lazzarini,R.A. (1982) J. Virol. 43, 166-173.
Schubert,M., Harminson,G.G. and Meier,H. (1984) J. Virol. 51, 505-514.
Shih,T.Y., Weeks,M.O., Young,H.A. and Scolnick,E.M. (1970) J. Virol. 31, 546-556.
Thomas,P.S. (1980) Proc. Natl. Acad. Sci. USA 77, 5201-5205.
Weck,P.K., Carroll,A.R., Shattuck,D.M. and Wagner,R.R. (1979) J. Virol. 30, 746-753.
Yelverton,E., Norton,S., Obijeski,J.F. and Goeddel,D.V. (1983) Science 219, 614-620.

REFERENCES

1. Sokol,F., Schlumberger,H.D., Wiktor,T.J. & Koprowski,H. (1969) Virology 38, 651-665.
2. Kurilla,M.G., Cabradilla,C.D., Holloway,B.P. & Keene,J.D. (1984) J. Virol. 50, 773-778.
3. Coslett,G.D., Holloway,B.P. & Obijeski,J.F. (1980) J. gen. Virol. 49, 161-180.
4. Holloway,B.P. & Obijeski,J.F. (1980) J. gen. Virol. 49, 181-195.
5. Flamand,A. & Delagneau,J.F. (1978) J. Virol. 36, 133-142.
6. Cox,J.F., Weiland,F., Dietzshold,B. & Schneider,L.G. (1981) in The replication of negative strand viruses, eds. Bishop,D.H.L. & Compans,R.W. (Elsevier, North-Holland, NY), pp. 639-645.
7. Delagneau,J.F., Perrin,P. & Atanatiu,P. (1981) Ann. Virol. (Inst.Pasteur) 132, 473-493.
8. Kawai,A. (1977) J. Virol. 24, 826-835.
9. Ermine,A. & Flamand,A. (1977) Ann. Microbiol. (Inst. Pasteur) 128, 477-488.
10. Rose,J.K. (1980) Cell 19, 415-421.
11. Gupta,K.C., & Kingsbury,D.W. (1984) Nucl. Acids Res. 12, 3829-3841.
12. Sanger,F., Nicklen,S. & Coulson,A.R. (1977) Proc. Natl. Acad. Sci. USA 74, 5463-5467.
13. Messing,J., Crea,R. & Seeburg,P.H. (1981) Nucl. Acids Res. 9, 309-321.
14. Kyte,J. & Doolittle,R.F. (1982) J. Mol. Biol. 157, 105-132.
15. Claverie,J.M., Sauvaget,I. & Bougueleret,L. (1985) Biochemie 67, 437-443.
16.Wilbur,W.J. & Lipman,D.J. (1983) Proc. Natl. Acad. Sci. USA 80, 726-730.
17. Gill,D.S. & Banerjee,A.K. (1985) J. Virol. 55, 60-66.
18. Bell,J.C & Prevec,L. (1985) J. Virol. 54, 697-702.
19. Lenard,J. & Compans, R.W. (1974) Biochem. Biophys. Acta 344, 51-94.
20. Wilson,T. & Lenard,J. (1981) Biochemistry 20, 1349-1354.
21. Capone,J. & Ghosh,H.P. (1984) Can. J. Biochem. Cell Biol. 62, 1174-1180.
22. Rose,J.K. and Gallione,C.J. (1981) J. Virol. 39, 519-528.
23. Wiktor,T.J., Gyorgy,E., Schlumberger,H.D., Sokol,F. & Koprowski,H. (1973) J. Immunol. 110, 269-276.
24. Anilionis,A., Wunner,W.H. & Curtis,P.J. (1981) Nature 294, 275-278.
25. Malek,L.T., Soostmeyer,G., Garvin,R.T. & James,E., (1984) in Modern Approaches to Vaccines, eds. Chanock,R.M. & Lerner,R.A. (Cold Spring Harbour Laboratory, Cold Spring Harbour, NY) pp. 203-208.
26. Yelverton,E., Norton,S., Obijeski,J.F. & Goeddel,D.V. (1983) Science 219, 614-620.
27. Lai,C.Y. & Dietzschold,B. (1981) Biochem. Biophys. Res. Com. 103, 536-542.

**Claims**

1. A DNA fragment which consists of the sequence extending from nucleotide 71 (ATG) to nucleotide 1 421 (TAA) of the nucleic acid shown in Figures 7 to 9 or of a portion of said sequence encoding a polypeptide having the same immunological properties as those of the nucleotide protein of the rabies virus.

2. A DNA fragment which consists of the sequence extending from nucleotide 1 514 to nucleotide 2 404 of the nucleic acid shown in figures 7 to 9 or of a portion of said sequence encoding a polypeptide having the same immunological properties as those of the M1 protein of the rabies virus.

3. A DNA fragment which consists of the sequence extending from nucleotide 2 496 to nucleotide 3 102 of the nucleic acid shown in figures 7 to 9 or of a portion of said sequence encoding a polypeptide having the same immunological properties as those of the M2 protein of the rabies virus.

4. A DNA fragment which comprises a sequence extending from nucleotide 2 496 to 3 102 of the nucleic acid shown in figures 7 to 9 or of a portion of said sequence encoding a polypeptide having the same immunological properties as those of the L protein of the rabies virus.

5. A vector containing a DNA fragment of any of said claims 1 to 4.

6. A probe for the detection of rabies virus RNA which comprises a DNA sequence contained in any of the DNA fragments of claims 1 to 4.

7. A purified polypeptide encoded by any one of the fragments of claims 1 to 4.

8. The antibodies which recognize the peptide of claim 7.

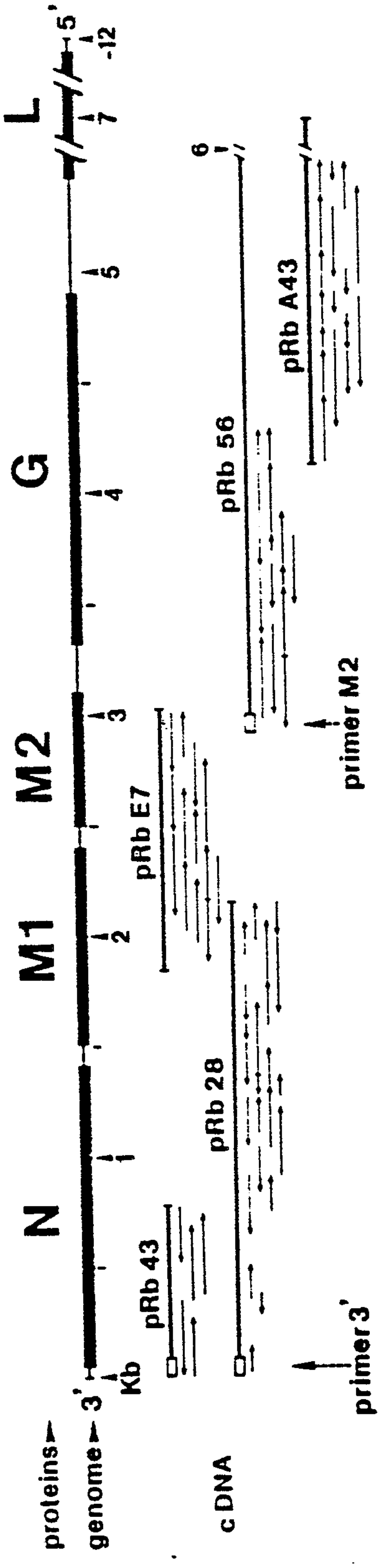
proteins
genome
3'
5'
N
M1
M2
G
L
Kb
1
2
3
4
5
6
7
-12
cDNA
pRb 43
pRb 28
pRb E7
pRb 56
pRb A43
primer 3'
primer M2

FIGURE 1

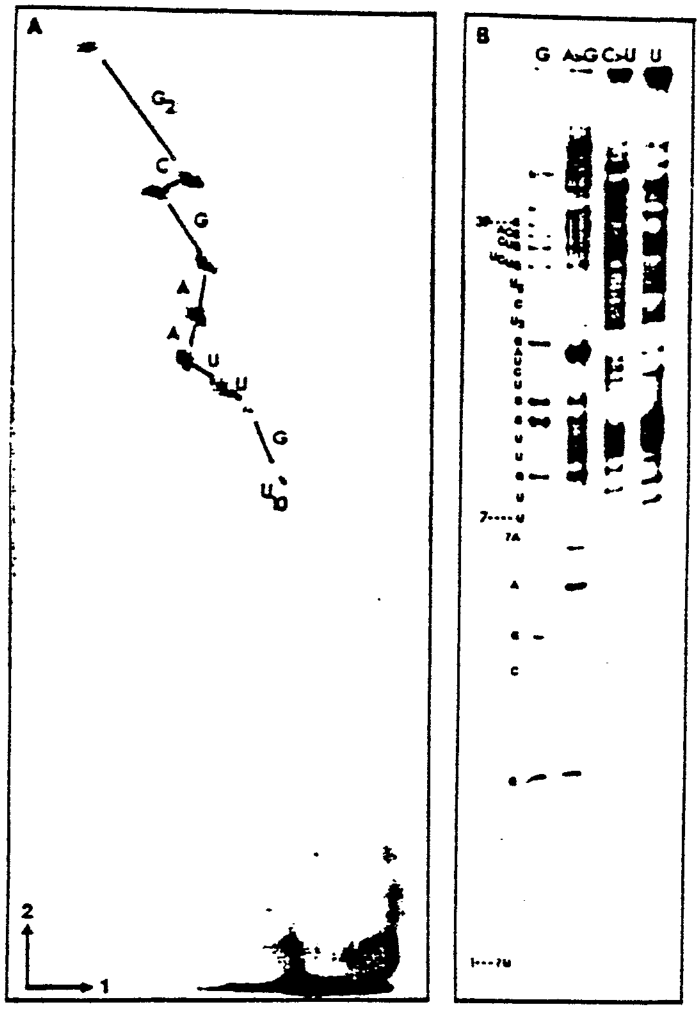


FIGURE 2

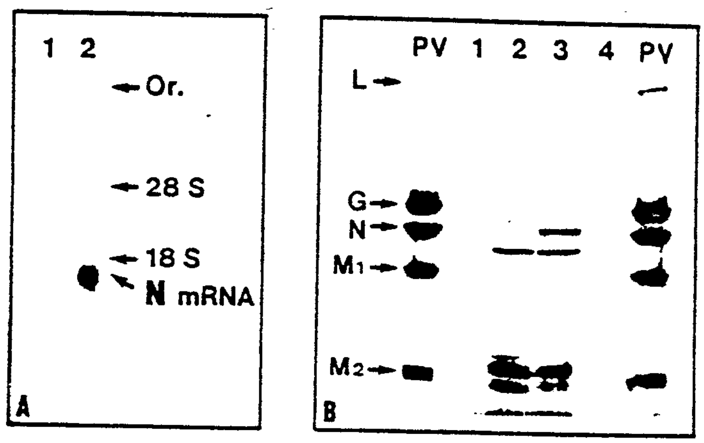


FIGURE 3

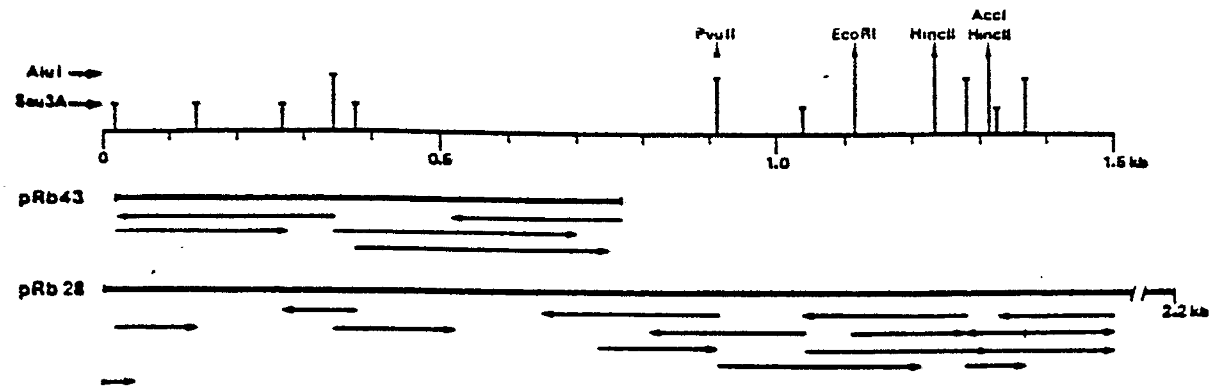


```
                A A C G    G   A                T   A                     C                                           G
1               | | | |  25|   |                |   | 60                  |            75                             |                 100
ACGCTTAACAACCAGATCAAAGAAAAAACAGACAGCGTCAATGGCAGAGCAAAAATGT AACACCTCTACA ATG GAT GCC GAC AAG ATT GTA TTC AAA GTC
Leader RNA                                                 N mRNA       Met Asp Ala Asp Lys Ile Val Phe Lys Val

                          125                                  150                                  G  175
AAT AAT CAG GTG GTC TCT TTG AAG CCT GAG ATT ATC GTG GAT CAA TAT GAG TAC AAG TAC CCT GCC ATC AAA GAT TTG AAA AAG
Asn Asn Gln Val Val Ser Leu Lys Pro Glu Ile Ile Val Asp Gln Tyr Glu Tyr Lys Tyr Pro Ala Ile Lys Asp Leu Lys Lys

                200                                   225                             250
CCC TGT ATA ACT CTA GGA AAG GCT CCC GAT TTA AAT AAA GCA TAC AAG TCA GTT TTA TCA TGC ATG AGC GCC GCC AAA CTT GAT
Pro Cys Ile Thr Leu Gly Lys Ala Pro Asp Leu Asn Lys Ala Tyr Lys Ser Val Leu Ser Cys Met Ser Ala Ala Lys Leu Asp

    275                             300                               325                                     350
CCT GAC GAT GTA TGT TCC TAT TTG GCG GCG GCA ATG CAG TTT TTT GAG GGG ACA TGT CCG GAA GAC TGG ACC AGC TAT GGA ATC
Pro Asp Asp Val Cys Ser Tyr Leu Ala Ala Ala Met Gln Phe Phe Glu Gly Thr Cys Pro Glu Asp Trp Thr Ser Tyr Gly Ile

                        375                            400                                     425
GTG ATT GCA CGA AAA GGA GAT AAG ATC ACC CCA GGT TCT CTG GTG GAG ATA AAA CGT ACT GAT GTA GAA GGG AAT TGG GCT CTG
Val Ile Ala Arg Lys Gly Asp Lys Ile Thr Pro Gly Ser Leu Val Glu Ile Lys Arg Thr Asp Val Glu Gly Asn Trp Ala Leu

            450                             475                             500
ACA GGA GGC ATG GAA CTG ACA AGA GAC CCC ACT GTC CCT GAG CAT GCG TCC TTA GTC GGT CTT CTC TTG AGT CTG TAT AGG TTG
Thr Gly Gly Met Glu Leu Thr Arg Asp Pro Thr Val Pro Glu His Ala Ser Leu Val Gly Leu Leu Leu Ser Leu Tyr Arg Leu

  525                           560                           575                                       600
AGC AAA ATA TCC GGG CAA AGC ACT GGT AAC TAT AAG ACA AAC ATT GCA GAC AGG ATA GAG CAG ATT TTT GAG ACA GCC CCT TTT
Ser Lys Ile Ser Gly Gln Ser Thr Gly Asn Tyr Lys Thr Asn Ile Ala Asp Arg Ile Glu Gln Ile Phe Glu Thr Ala Pro Phe

                      625                              650                             675
GTT AAA ATC GTG GAA CAC CAT ACT CTA ATG ACA ACT CAC AAA ATG TGT GCT AAT TGG AGT ACT ATA CCA AAC TTC AGA TTT TTG
Val Lys Ile Val Glu His His Thr Leu Met Thr Thr His Lys Met Cys Ala Asn Trp Ser Thr Ile Pro Asn Phe Arg Phe Leu

            700                                 725                         750
GCC GGA ACC TAT GAC ATG TTT TTC TCC CGG ATT GAG CAT CTA TAT TCA GCA ATC AGA GTG GGC ACA GTT GTC ACT GCT TAT GAA
Ala Gly Thr Tyr Asp Met Phe Phe Ser Arg Ile Glu His Leu Tyr Ser Ala Ile Arg Val Gly Thr Val Val Thr Ala Tyr Glu

775                           800                                 825                                 850
GAC TGT TCA GGA CTG GTG TCA TTT ACT GGG TTC ATA AAA CAA ATC AAT CTC ACC GCT AGA GAG GCA ATA CTA TAT TTC TTC CAC
Asp Cys Ser Gly Leu Val Ser Phe Thr Gly Phe Ile Lys Gln Ile Asn Leu Thr Ala Arg Glu Ala Ile Leu Tyr Phe Phe His

                    875                                900                                   925
AAG AAC TTT GAG GAA GAG ATA AGA AGA ATG TTT GAG CCA GGG CAG GAG ACA GCT GTT CCT CAC TCT TAT TTC ATC CAC TTC CGT
Lys Asn Phe Glu Glu Glu Ile Arg Arg Met Phe Glu Pro Gly Gln Glu Thr Ala Val Pro His Ser Tyr Phe Ile His Phe Arg

          950                        975                                    1000                                1025
TCA CTA GGC TTG AGT GGG AAA TCT CCT TAT TCA TCA AAT GCT GTT GGT CAC GTG TTC AAT CTC ATT CAC TTT GTA GGA TGC TAT
Ser Leu Gly Leu Ser Gly Lys Ser Pro Tyr Ser Ser Asn Ala Val Gly His Val Phe Asn Leu Ile His Phe Val Gly Cys Tyr

                           1050                                1075                                  1100
ATG GGT CAA GTC AGA TCC CTA AAT GCA ACG GTT ATT GCT GCA TGT GCT CCT CAT GAA ATG TCT GTT CTA GGG GGC TAT CTG GGA
Met Gly Gln Val Arg Ser Leu Asn Ala Thr Val Ile Ala Ala Cys Ala Pro His Glu Met Ser Val Leu Gly Gly Tyr Leu Gly

                1125                                 1150                                1175
GAG GAA TTC TTC GGG AAA GGG ACA TTT GAA AGA AGA TTC TTC AGA GAT GAG AAA GAA CTT CAA GAA TAC GAG GCG GCT GAA CTG
Glu Glu Phe Phe Gly Lys Gly Thr Phe Glu Arg Arg Phe Phe Arg Asp Glu Lys Glu Leu Gln Glu Tyr Glu Ala Ala Glu Leu

    1200                              1225                                 1250                                1275
ACA AAG ACT GAC GTA GCA CTG GCA GAT GAT GGA ACT GTC AAC TCT GAC GAC GAG GAC TAC TTC TCA GGT GAA ACC AGA AGT CCG
Thr Lys Thr Asp Val Ala Leu Ala Asp Asp Gly Thr Val Asn Ser Asp Asp Glu Asp Tyr Phe Ser Gly Glu Thr Arg Ser Pro

                        1300                                 1325                               1350
GAA GCT GTT TAT ACT CGA ATC ATA ATG AAT GGA GGT CGA CTG AAG AGA TCG CAC ATA CGG AGA TAT GTC TCA GTC AGT TCC AAT
Glu Ala Val Tyr Thr Arg Ile Ile Met Asn Gly Gly Arg Leu Lys Arg Ser His Ile Arg Arg Tyr Val Ser Val Ser Ser Asn

              1375                             1400                               1425                          1450
CAT CAA GCT CGT CCA AAC TCA TTC GCC GAG TTT CTA AAC AAG ACA TAT TCG AGT GAC TCA TAA GAAGTTGAATAACAAAATGCCGGAAAT
His Gln Ala Arg Pro Asn Ser Phe Ala Glu Phe Leu Asn Lys Thr Tyr Ser Ser Asp Ser ***

                   1475                    1500
CTACGGATTGTGTATATCCATCATGAAAAAAA CT AACACCCCTCCTTTCG...
                         N mRNA      M1 mRNA
```

FIGURE 4

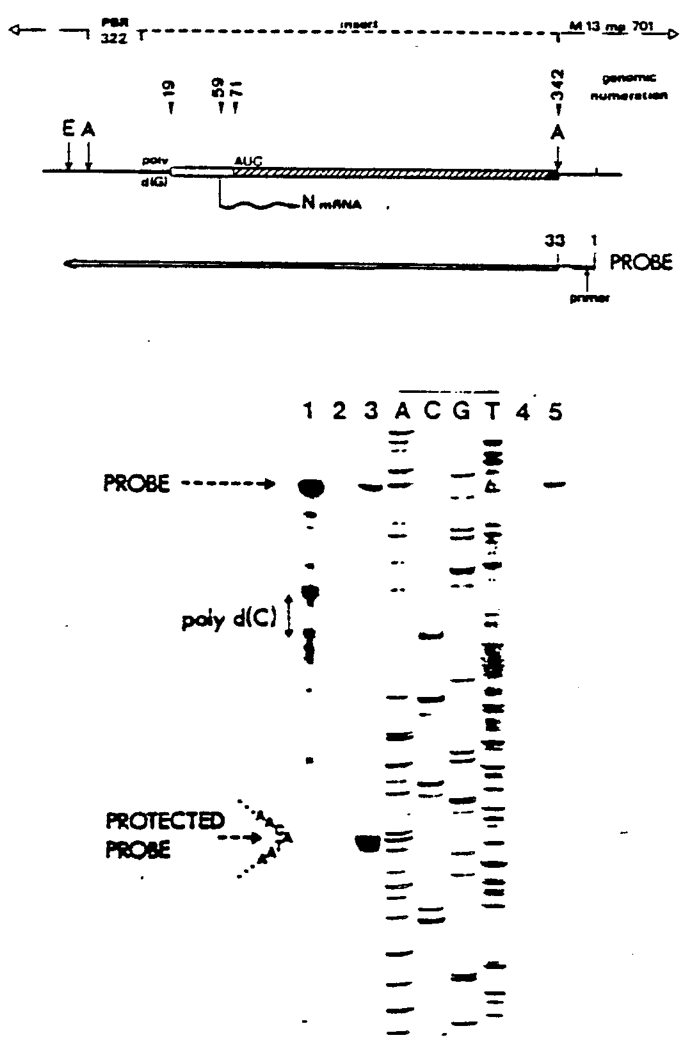
PBR
322
insert
M 13 mp 701
19
59
71
342
genomic
numeration
E A
A
poly
d(G)
AUG
N mRNA
33
1
PROBE
primer
1 2 3 A C G T 4 5
PROBE
poly d(C)
PROTECTED
PROBE

FIGURE 5

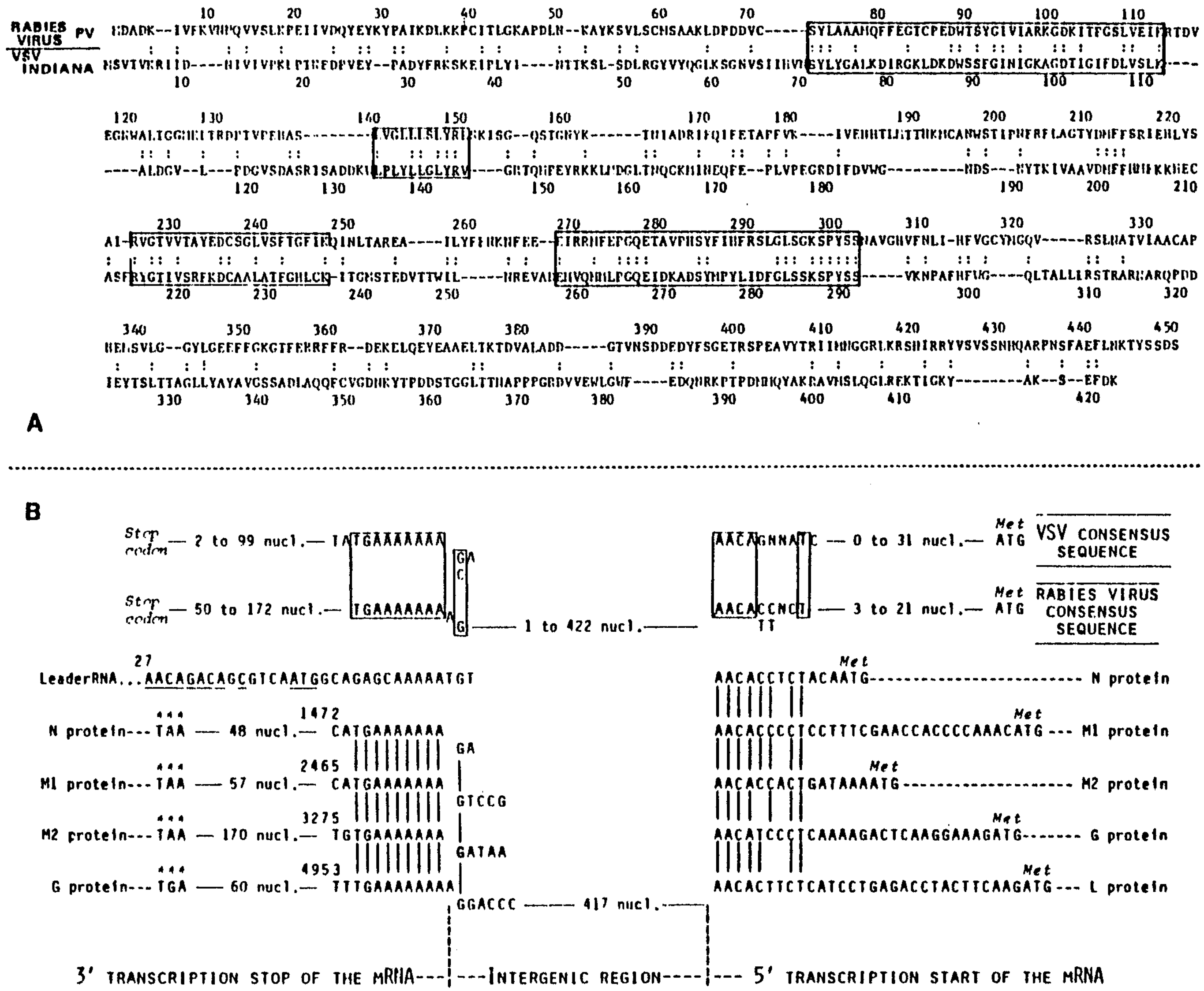
RABIES VIRUS PV
VSV INDIANA
A
B
Stop codon — 2 to 99 nucl.—
Stop codon — 50 to 172 nucl.—
1 to 422 nucl.
— 0 to 31 nucl.— Met ATG
— 3 to 21 nucl.— Met ATG
VSV CONSENSUS SEQUENCE
RABIES VIRUS CONSENSUS SEQUENCE
LeaderRNA...AACAGACAGCGTCAATGGCAGAGCAAAAATGT
N protein---TAA — 48 nucl.— CATGAAAAAAA
M1 protein---TAA — 57 nucl.— CATGAAAAAAA
M2 protein---TAA — 170 nucl.— TGTGAAAAAAA
G protein---TGA — 60 nucl.— TTTGAAAAAAAA
GA
GTCCG
GATAA
GGACCC — 417 nucl.
AACACCTCTACAATG---- N protein
AACACCCCTCCTTTCGAACCACCCCAAACATG --- M1 protein
AACACCACTGATAAAATG---- M2 protein
AACATCCCTCAAAAGACTCAAGGAAAGATG---- G protein
AACACTTCTCATCCTGAGACCTACTTCAAGATG --- L protein
3' TRANSCRIPTION STOP OF THE mRNA
INTERGENIC REGION
5' TRANSCRIPTION START OF THE mRNA

FIGURE 6

FIGURE 7

►LEADER RNA | N ► MetAspAlaAspLysIleValPheLysValAsnAsnGlnValValSerLeu

ACGCTTAACAACCAGATCAAAGAAAAAACAGACAGCGTCAATGGCAGAGCAAAAATGTAACACCTCTACAATGGATGCCGACAAGATTGTATTCAAAGTCAATAATCAGGTGGTCTCTTT

100

LysProGluIleIleValAspGlnTyrGluTyrLysTyrProAlaIleLysAspLeuLysLysProCysIleThrLeuGlyLysAlaProAspLeuAsnLysAlaTyrLysSerValLeu

GAAGCCTGAGATTATCGTGGATCAATATGAGTACAAGTACCCTGCCATCAAAGATTTGAAAAAGCCCTGTATAACTCTAGGAAAGGCTCCCGATTTAAATAAAGCATACAAGTCAGTTTT

200

SerCysMetSerAlaAlaLysLeuAspProAspAspValCysSerTyrLeuAlaAlaAlaMetGlnPhePheGluGlyThrCysProGluAspTrpThrSerTyrGlyIleValIleAla

ATCATGCATGAGCGCCGCCAAACTTGATCCTGACGATGTATGTTCCTATTTGGCGGCGGCAATGCAGTTTTTTGAGGGCACATGTCCGGAAGACTGGACCAGCTATGGAATCGTGATTGC

300

ArgLysGlyAspLysIleThrProGlySerLeuValGluIleLysArgThrAspValGluGlyAsnTrpAlaLeuThrGlyGlyMetGluLeuThrArgAspProThrValProGluHis

ACGAAAAGGAGATAAGATCACCCCAGGTTCTCTGGTGGAGATAAAACGTACTGATGTAGAAGGGAATTGGGCTCTGACAGGAGGCATGGAACTGACAAGAGACCCCACTGTCCCTGAGCA

400

AlaSerLeuValGlyLeuLeuLeuSerLeuTyrArgLeuSerLysIleSerGlyGlnSerThrGlyAsnTyrLysThrAsnIleAlaAspArgIleGluGlnIlePheGluThrAlaPro

TGCGTCCTTAGTCGGTCTTCTCTTGAGTCTGTATAGGTTGAGCAAAATATCCGGGCAAAGCACTGGTAACTATAAGACAAACATTGCAGACAGGATAGAGCAGATTTTTGAGACAGCCCC

500 600

PheValLysIleValGluHisHisThrLeuMetThrThrHisLysMetCysAlaAsnTrpSerThrIleProAsnPheArgPheLeuAlaGlyThrTyrAspMetPhePheSerArgIle

TTTTGTTAAAATCGTGGAACACCATACTCTAATGACAACTCACAAAATGTGTGCTAATTGGAGTACTATACCAAACTTCAGATTTTTGGCCGGAACCTATGACATGTTTTTCTCCCGGAT

700

GluHisLeuTyrSerAlaIleArgValGlyThrValValThrAlaTyrGluAspCysSerGlyLeuValSerPheThrGlyPheIleLysGlnIleAsnLeuThrAlaArgGluAlaIle

TGAGCATCTATATTCAGCAATCAGAGTGGGCACAGTTGTCACTGCTTATGAAGACTGTTCAGGACTGGTGTCATTTACTGGGTTCATAAAACAAATCAATCTCACCGCTAGAGAGGCAAT

800

LeuTyrPhePheHisLysAsnPheGluGluGluIleArgArgMetPheGluProGlyGlnGluThrAlaValProHisSerTyrPheIleHisPheArgSerLeuGlyLeuSerGlyLys

ACTATATTTCTTCCACAAGAACTTTGAGGAAGAGATAAGAAGAATGTTTGAGCCAGGGCAGGAGACAGCTGTTCCTCACTCTTATTTCATCCACTTCCGTTCACTAGGCTTGAGTGGCAA

900

SerProTyrSerSerAsnAlaValGlyHisValPheAsnLeuIleHisPheValGlyCysTyrMetGlyGlnValArgSerLeuAsnAlaThrValIleAlaAlaCysAlaProHisGlu

ATCTCCTTATTCATCAAATGCTGTTGGTCACGTGTTCAATCTCATTCACTTTGTAGGATGCTATATGGGTCAAGTCAGATCCCTAAATGCAACGGTTATTGCTGCATGTGCTCCTCATGA

1000

MetSerValLeuGlyGlyTyrLeuGlyGluGluPhePheGlyLysGlyThrPheGluArgArgPhePheArgAspGluLysGluLeuGlnGluTyrGluAlaAlaGluLeuThrLysThr

AATGTCTGTTCTAGGGGGCTATCTGGGAGAGGAATTCTTCGGGAAAGGAACATTTGAAAGAAGATTCTTCAGAGATGAGAAAGAACTTCAAGAATACGAGGCGGCTGAACTGACAAAGAC

1100 1200

AspValAlaLeuAlaAspAspGlyThrValAsnSerAspAspGluAspTyrPheSerGlyGluThrArgSerProGluAlaValTyrThrArgIleIleMetAsnGlyGlyArgLeuLys

TGACGTAGCACTGGCAGATGATGGAACTGTCAACTCTGACGACGAGGACTACTTCTCAGGTGAAACCAGAAGTCCGGAAGCTGTTTATACTCGAATCATAATGAATGGAGGTCGACTGAA

1300

ArgSerHisIleArgArgTyrValSerValSerSerAsnHisGlnAlaArgProAsnSerPheAlaGluPheLeuAsnLysThrTyrSerSerAspSer***

GAGATCGCACATACGGAGATATGTCTCAGTCAGTTCCAATCATCAAGCTCGTCCAAACTCATTCGCCGAGTTTCTAAACAAGACATATTCGAGTGACTCATAAGAAGTTGAATAACAAAA

1400

↓↓ M1 ► MetSerLysIlePheValAsnProSerAlaIleArgAlaGlyLeuAla

TGCCGGAAATCTACGGATTGTGTATATCCATCATGAAAAAAACTAACACCCCTCCTTTCGAACCACCCCAAACATGAGCAAGATCTTTGTCAATCCTAGTGCTATTAGAGCCGGTCTGGC

1500

AspLeuGluMetAlaGluGluThrValAspLeuIleAsnArgAsnIleGluAspAsnGlnAlaHisLeuGlnGlyGluProIleGluValAspAsnLeuProGluAspMetGlyArgLeu

CGATCTTGAGATGGCTGAAGAAACTGTTGATCTGATCAATAGAAATATCGAAGACAATCAGGCTCATCTCCAAGGGGAACCCATAGAAGTGGACAATCTCCCTGAGGATATGGGGCGACT

1600

HisLeuAspAspGlyLysSerProAsnProGlyGluMetAlaLysValGlyGluGlyLysTyrArgGluAspPheGlnMetAspGluGlyGluAspProSerLeuLeuPheGlnSerTyr

TCACCTGGATGATGGAAAATCGCCCAACCCTGGTGAGATGGCCAAGGTGGGAGAAGGCAAGTATCGAGAGGACTTTCAGATGGATGAAGGAGAGGATCCTAGCCTCCTGTTCCAGTCATA

1700 1800

LeuAspAsnValGlyValGlnIleValArgGlnIleArgSerGlyGluArgPheLeuLysIleTrpSerGlnThrValGluGluIleIleSerTyrValAlaValAsnPheProAsnPro

CCTGGACAATGTTGGAGTCCAAATAGTCAGACAAATAAGGTCAGGAGAGAGATTTCTCAAGATATGGTCACAGACCGTAGAAGAGATTATATCCTATGTCGCGGTCAACTTTCCCAACCC

1900

FIGURE 8

```
ProGlyLysSerSerGluAspLysSerThrGlnThrThrGlyArgGluLeuLysLysGluThrThrProThrProSerGlnArgGluSerGlnSerSerLysAlaArgMetAlaAlaGln
TCCAGGAAAGTCTTCAGAGGATAAATCAACCCAGACTACCGGCCGAGAGCTCAAGAAGGAGACAACACCCACTCCTTCTCAGAGAGAAAGCCAATCCTCGAAAGCCAGGATGGCGGCTCA
                                                                                     2000
ThrAlaSerGlyProProAlaLeuGluTrpSerAlaThrAsnGluGluAspAspLeuSerValGluAlaGluIleAlaHisGlnIleAlaGluSerPheSerLysLysTyrLysPhePro
AACTGCTTCTGGCCCTCCAGCCCTTGAATGGTCGGCCACCAATGAAGAGGATGATCTATCAGTGGAGGCTGAGATCGCTCACCAGATTGCAGAAAGTTTCTCCAAAAAATATAAGTTTCC
                                                          2100
SerArgSerSerGlyIleLeuLeuTyrAsnPheGluGlnLeuLysMetAsnLeuAspAspIleValLysGluAlaLysAsnValProGlyValThrArgLeuAlaArgAspGlySerLys
CTCTCGATCCTCAGGGATACTCTTGTATAATTTTGAGCAATTGAAAATGAACCTTGATGATATAGTTAAAGAGGCAAAAAATGTACCAGGTGTGACCCGTTTAGCCCGTGACGGGTCCAA
                                     2200
LeuProLeuArgCysValLeuGlyTrpValAlaLeuAlaAsnSerLysLysPheGlnLeuLeuValGluSerAsnLysLeuSerLysIleMetGlnAspAspLeuAsnArgTyrThrSer
ACTCCCCCTAAGATGTGTACTGGGATGGGTCGCCTTGGCCAACTCTAAGAAATTCCAGTTGTTAGTCGAATCCAACAAGCTGAGTAAAATCATGCAAGATGACTTGAATCGCTATACATC
              2300                                                                                                    2400
Cys***                                                                                      M2 ► MetAsnPheLeuArgLysIleValLys
TTGCTAACCGAACCTCTCCACTCAGTCCCTCTAGACAATAAAGTCCGAGATGTCCTAAAGTCAACATGAAAAAAACAGGCAACACCACTGATAAAATGAACTTTCTACGTAAGATAGTGA
                                                                                                       2500
AsnCysArgAspGluAspThrGlnLysProSerProValSerAlaProLeuAspAspAspAspLeuTrpLeuProProProGluTyrValProLeuLysGluLeuThrSerLysLysAsn
AAAATTGCAGGGACGAGGACACTCAAAAACCCTCTCCCGTGTCAGCCCCTCTGGATGACGATGACTTGTGGCTTCCACCCCCTGAATACGTCCCGCTAAAAGAACTTACAAGCAAGAAGA
                                                                                                2600
ArgArgAsnPheCysIleAsnGlyGlyValLysValCysSerProAsnGlyTyrSerPheGlyIleLeuArgHisIleLeuArgSerPheAspGluIleTyrSerGlyAsnHisArgMet
ACAGGAGGAACTTTTGTATCAACGGAGGGGTTAAAGTGTGTAGCCCGAATGGTTACTCGTTCGGGATCCTGCGGCACATTCTGAGATCATTCGACGAGATATATTCTGGGAATCATAGGA
                                                              2700
ValGlyLeuValLysValValIleGlyLeuAlaLeuSerGlyAlaProValProGluGlyMetAsnTrpValTyrLysLeuArgArgThrLeuIlePheGlnTrpAlaAspSerArgGly
TGGTCGGGTTAGTCAAAGTAGTTATTGGACTGGCTTTGTCAGGAGCTCCAGTCCCTGAGGGCATGAACTGGGTATACAAGTTGAGGAGAACCCTTATCTTCCAGTGGGCTGATTCCAGGG
                                     2800
ProLeuGluGlyGluGluLeuGluTyrSerGlnGluIleThrTrpAspAspAsnThrGluPheValGlyLeuGlnIleArgValSerAlaLysGlnCysHisIleArgGlyArgIleTrp
GCCCTCTTGAAGGGGAGGAGTTGCAATACTCTCAGGAGATCACTTGGGATGATAATACTGAGTTCGTCGGATTGCAAATAAGAGTGAGTGCAAAACAGTGTCATATCCGGGGCAGAATCT
              2900                                                                                                    3000
CysIleAsnMetAsnSerArgAlaGlyGlnLeuTrpSerAspMetSerLeuGlnThrGlnArgSerGluGluAspLysAspSerSerLeuLeuLeuGlu***
GGTGTATCAACATGAACTCGAGAGCAGGTCAACTATGGTCTGACATGTCTCTTCAGACACAAAGGTCCGAAGAGGACAAAGATTCCTCTCTGCTTCTAGAATAATCAGATTATATCCCGC
                                                                                                 3100
AAATTTATCACTTGTTTACCTCTGGAGGAGAGAACATATGGCTCAACTCCAACCCTTGGGGGCAATATAACAAAAAACATGTTATGCTGCCATTAAACCGCTGCATTTCATCAAAGTC
                                                                                             3200
                                                                         CVS     - - - - Val - - - - - Leu - - Gly - Ser
                                                                         ERA     - - - - - - - - - - - - - - - - - - -
                                                                         PV  G ► MetValProGlnAlaLeuLeuPheValProLeuLeuValPhePro
AAGTTAATTACCTTTACATTTTGATCCTCTTGCATGTGAAAAAAACTATTAACATCCCTCAAAAGACTCAAGGAAAGATGGTTCCTCAGGCTCTCCTGTTTGTACCCCTTCTGGTTTTTC
                                                               3300
- - - - - - - - - - - - - - - - - - - - - - - - - - Arg - - - - - - - - - - - - -
- - - - - - - - - - - - - - - - - - - - - - - - - - - - - - - - - - - - - - - - -
LeuCysPheGlyLysPheProIleTyrThrIleProAspLysLeuGlyProTrpSerProIleAspIleHisHisLeuSerCysProAsnAsnLeuValValGluAspGluGlyCysThr
CATTGTGTTTTGGGAAATTCCCTATTTACACCATACCAGACAAGCTTGGTCCCTGGAGCCCCGATTGACATACATCACCTCAGCTGCCCAAACAATTTGGTAGTGGAGGACGAAGGATGCA
                                     3400
```

FIGURE 9

```
AsnLeuSerGlyPheSerTyrMetGluLeuLysValGlyTyrIleSerAlaIleLysMetAsnGlyPheThrCysThrGlyValValThrGluAlaGluThrTyrThrAsnPheValGly
CCAACCTGTCAGGGTTCTCCTACATGGAACTTAAAGTTGGATACATCTCAGCCATAAAAATGAACGGGTTCACTTGCACAGGCGTTGTGACGGAGGCTGAAACCTACACTAACTTCGTTG
          3500                                                                                                      3600
TyrValThrThrThrPheLysArgLysHisPheArgProThrProAspAlaCysArgAlaAlaTyrAsnTrpLysMetAlaGlyAspProArgTyrGluGluSerLeuHisAsnProTyr
GTTATGTCACAACCACGTTCAAAAGAAAGCATTTCCGCCCAACACCAGATGCATGTAGAGCCGCGTACAACTGGAAGATGGCCGGTGACCCCAGATATGAAGAGTCTCTACACAATCCGT
                                                                                                            3700
ProAspTyrHisTrpLeuArgThrValLysThrThrLysGluSerLeuValIleIleSerProSerValAlaAspLeuAspProTyrAspArgSerLeuHisSerArgValPheProGly
ACCCTGACTACCACTGGCTTCGAACTGTAAAAACCACCAAGGAGTCTCTCGTTATCATATCTCCAAGTGTGGCAGATTTGGACCCATATGACAGATCCCTTCACTCGAGGGTCTTCCCTG
                                                                                           3800
GlyAsnCysSerGlyValAlaValSerSerThrTyrCysSerThrAsnHisAspTyrThrIleTrpMetProGluAsnProArgLeuGlyMetSerCysAspIlePheThrAsnSerArg
GCGGGAATTGCTCAGGAGTAGCGGTGTCTTCTACCTACTGCTCCACTAACCACGATTACACCATTTGGATGCCCGAGAATCCGAGACTAGGGATGTCTTGTGACATTTTTACCAATAGTA
                                                                      3900
GlyLysArgAlaSerLysGlySerGluThrCysGlyPheValAspGluArgGlyLeuTyrLysSerLeuLysGlyAlaCysLysLeuLysLeuCysGlyValLeuGlyLeuArgLeuMet
GAGGGAAGAGAGCATCCAAAGGGAGTGAGACTTGCGGCTTTGTAGATGAAAGAGGCCTATATAAGTCTTTAAAAGGAGCATGCAAACTCAAGTTATGTGGAGTTCTAGGACTTAGACTTA
                                                   4000
AspGlyThrTrpValAlaMetGlnThrSerAsnGluThrLysTrpCysProProGlyGlnLeuValAsnLeuHisAspPheArgSerAspGluIleGluHisLeuValValGluGluLeu
TGGATGGAACATGGGTCGCGATGCAAACATCAAATGAAACCAAATGGTGCCCTCCCGGTCAGTTGGTGAATTTGCACGACTTTCGCTCAGACGAAATTGAGCACCTTGTTGTAGAGGAGT
                                                                                                                   4200
          4100
ValLysLysArgGluGluCysLeuAspAlaLeuGluSerIleMetThrThrLysSerValSerPheArgArgLeuSerHisLeuArgLysLeuValProGlyPheGlyLysAlaTyrThr
TGGTCAAGAAGAGAGAGGAGTGTCTGGATGCACTAGAGTCCATCATGACCACCAAGTCAGTGAGTTTCAGACGTCTCAGTCATTTAAGAAAACTTGTCCCTGGGTTTGGAAAAGCATATA
                                                                                                          4300
IlePheAsnLysThrLeuMetGluAlaAspAlaHisTyrLysSerValArgThrTrpAsnGluIleIleProSerLysGlyCysLeuArgValGlyGlyArgCysHisProHisValAsn
CCATATTCAACAAGACCTTGATGGAAGCCGATGCTCACTACAAGTCAGTCAGAACTTGGAATGAGATCATCCCTTCAAAAGGGTGTTTAAGAGTTGGGGGGAGGTGTCATCCTCATGTAA
                                                                                            4400
GlyValPhePheAsnGlyIleIleLeuGlyProAspGlyAsnValLeuIleProGluMetGlnSerSerLeuLeuGlnGlnHisMetGluLeuLeuValSerSerValIleProLeuMet
ACGGGGTATTTTTCAATGGTATAATATTAGGACCTGACGGCAATGTCTTAATCCCAGAGATGCAATCATCCCTCCTCCAGCAACATATGGAGTTGTTGGTATCCTCGGTTATCCCCCTTA
                                                                          4500
HisProLeuAlaAspProSerThrValPheLysAsnGlyAspGluAlaGluAspPheValGluValHisLeuProAspValHisGluArgIleSerGlyValAspLeuGlyLeuProAsn
TGCACCCCCTGGCAGACCCGTCTACCGTTTTCAAGAACGGTGACGAGGCTGAGGATTTTGTTGAAGTTCACCTTCCCGATGTGCACGAACGGATCTCAGGAGTTGACTTGGGTCTCCCGA
                                                  4600
TrpGlyLysTyrValLeuLeuSerAlaGlyAlaLeuThrAlaLeuMetLeuIleIlePheLeuMetThrCysTrpArgArgValAsnArgSerGluProThrGlnHisAsnLeuArgGly
ACTGGGGGAAGTATGTATTACTGAGTGCAGGGGCCCTGACTGCCTTGATGTTGATAATTTTCCTGATGACATGCTGGAGAAGAGTCAATCGATCGGAACCTACACAACACAATCTCAGAG
                                                                                                                    4800
          4700
ThrGlyArgGluValSerValThrProGlnSerGlyLysIleIleSerSerTrpGluSerTyrLysSerGlyGlyGluThrGlyLeu***
GGACAGGGAGGGAGGTGTCAGTCACTCCCCAAAGCGGGAAGATCATATCTTCATGGGAATCATACAAGAGCGGGGGTGAGACCGGACTGTGAGAGCTGGCCCGTCCTTTCAACGATCCAAG
                                                                                                          4900
TCCTGAAGATCACCTCCCCTTGGGGGGTTCTTTTTGAAAAAAAACCTGGGTTCAATAGTCCTCCTTGAACTCCATGCAACTGGGTAGATTCAAGAGTCATGAGATTTTCATTAATCCTCT
                                                                                     5000
CAGTTGATCAAGCAAGATCATGTAGATTCTCATAATAGGGGAGATCTTCTAGCAGTTTCAGTGACTAACGGTGCTTTCATTCTCCAGGAACTGACACCAACAGTTCTAGACAAATCACGG
                                                                 5100
GGTGTCTCAGGTGATTCTGCCCTTGGGCACAGACAAAGGTCATGGTGTGTTCCATGATAGCCGGACTCAGGATGAGTTAATTGAGAGAGGCAATCTTCCTCCCGTGAAGGACACAAGCAGT
                                           5200
AGCTCACAATCATCTCGTGTTTCAGCAAAGTGTGCATAATTATAAAGTGCTGGGTCATCTAAGCTTTTCAGTCGAGAAAAAAACAGTAGATCAGAAGAACAACTGGCAACACTTCTCATC
                                                                                                                    5400
          5300
      L ► MetLeuAspProGlyGluValTyrAspAspProIleAspProIleGluLeuGluAlaGluProArgGlyThrProThrValPro
CTGAGACCTACTTCAAGATGCTCGATCCTGGAGAGGTCTATGATGACCCTATTGACCCAATCGAGTTAGAGGCTGAACCCAGAGGAACCCCCACTGTCCC
                                                                                                    5500
```

European Patent Office

# EUROPEAN SEARCH REPORT

Application number

EP 86 40 0576

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DEV. CELL. BIOL., vol. 7, 1981, pages 721-725, Elsevier North Holland Inc., Amsterdam, NL; P.J. CURTIS et al.: "Cloning of full length cDNA from the rabies virus glycoprotein gene" * Whole document * | 1 | C 12 N 15/00<br>C 12 P 21/02<br>A 61 K 39/205<br>C 12 Q 1/70<br>C 07 K 15/00 |
| Y | Idem | 2-6 | |
| | --- | | |
| X | CHEMICAL ABSTRACTS, vol. 93, no. 9, 1st September 1980, page 261, abstract no. 232921z, Columbus, Ohio, US; W.H. WUNNER et al.: "Rabies mRNA translation in Xenopus laevis oocytes", & J. VIROL. 1980, 36(1), 133-42 * Whole abstract * | 1,7 | |
| | | | **TECHNICAL FIELDS SEARCHED (Int. Cl.4)** |
| Y | Idem | 2-6 | C 12 N<br>C 12 P<br>A 61 K |
| | --- | | |
| X | CHEMICAL ABSTRACTS, vol. 93, no. 21, 24th November 1980, page 200, abstract no. 90324f, Columbus, Ohio, US; D. PENNICA et al.: "In vitro translation of rabies virus messenger RNAs", & VIROLOGY 1980, 103(2), 517-21 * Whole abstract * | 7 | |
| Y | Idem | 1-6 | |
| | --- -/- | | |

The present search report has been drawn up for all claims

| Place of search | Date of completion of the search | Examiner |
|---|---|---|
| THE HAGUE | 16-10-1986 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number

EP 86 40 0576

Page 2

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| D,X | CHEMICAL ABSTRACTS, vol. 93, no. 25, 22nd December 1980, page 353, abstract no. 200797x, Columbus, Ohio, US; G.D. COSLETT et al.: "The structural proteins of rabies virus and evidence for their synthesis from separate monocistronic RNA species", & J. GEN. VIROL. 1980, 49(1), 161-80<br>* Whole abstract *<br>--- | 7 | |
| D,Y | CHEMICAL ABSTRACTS, vol. 93, no. 21, 24th November 1980, page 354, abstract no. 200798y, Columbus, Ohio, US; B.P. HOLLOWAY et al.: "Rabies virus-induced RNA synthesis in BHK21 cells", & J. GEN. VIROL. 1980, 49(1), 181-95<br>* Whole abstract *<br>--- | 1-6 | TECHNICAL FIELDS SEARCHED (Int. Cl.4) |
| Y | CHEMICAL ABSTRACTS, vol. 90, no. 3, 15th January 1979, page 298, abstract no. 18925y, Columbus, Ohio, US; A. FLAMAND et al.: "Transcriptional mapping of rabies virus in vivo", & J. VIROL. 1978, 28(2), 518-23<br>* Whole abstract *<br>--- | 1-6 | |
| Y | FR-A-2 515 685 (THE WISTAR INSTITUTE)<br>* Whole document *<br>--- -/- | 1-6 | |

The present search report has been drawn up for all claims

| Place of search | Date of completion of the search | Examiner |
|---|---|---|
| THE HAGUE | 16-10-1986 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent Office

# EUROPEAN SEARCH REPORT

Application number

EP 86 40 0576



## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 117 657 (GENENTECH INC.)<br>* Whole document *<br>--- | 1-6 | |
| D,A | CHEMICAL ABSTRACTS, vol. 101, no. 11, 10th September 1984, page 131, abstract no. 84727g, Columbus, Ohio, US; M.G. KURILLA et al.: "Nucleotide sequence and host La protein interactions of rabies virus leader RNA", & J. VIROL. 1984, 50(3), 773-8<br>* Whole abstract *<br>----- | 1-6 | |

TECHNICAL FIELDS SEARCHED (Int. Cl.4)

The present search report has been drawn up for all claims

| Place of search | Date of completion of the search | Examiner |
|---|---|---|
| THE HAGUE | 16-10-1986 | DESCAMPS J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82